# EUROPEAN PATENT SPECIFICATION

(11) **EP 1 736 116 B1**
(45) Date of publication and mention of the grant of the patent: **18.11.2009**
(21) Application number: 06253225.4
(22) Date of filing: 22.06.2006
(51) Int. Cl.: A61F 2/06

(54) **Graft for aneurysm repair**
Transplantat zur Behebung eines Aneurysmas
Greffe pour la réparation d'un anévrisme

(30) Priority: 23.06.2005 GB 0512786
(43) Date of publication of application: 27.12.2006
(73) Proprietor: VASCUTEK LIMITED, Renfrewshire, Scotland PA4 9RR (GB)
(72) Inventor: Neri, Eugenio, 53100 Siena (IT)
(74) Representative: Ouzman, Beverley Nicola Claire

(56) References cited:
- EP-A- 1 245 202
- WO-A1-98/06355
- FR-A- 2 850 008
- US-A- 5 957 973
- NERI E. ET AL: 'The "Elephant trunk" technique made easier' ANN. THORAC. SURG. no. 78, 2004, pages 17 - 18, XP008090879

## Description

The present application concerns improvements to a graft used to treat aneurysmal disease.

Aortic aneurysm is characterised by the dilation or ballooning out of part of the wall of the aorta, the artery through which blood flows out of the heart to the body. The majority of aortic aneurysms cause little or no symptoms and small aneurysms can be controlled by a reduction in blood pressure achieved through the administration of beta blockers. However, larger aneurysms pose significant dangers to the patient and may require surgery to prevent rupture, which is frequently fatal. An aneurysm may be caused due to genetic conditions or other diseases and forms where the wall of the aorta is weakened, often due to the build up of plaque. High blood pressure can also increase the likelihood of aneurysm development. Rupture of an aortic aneurysm gives only a 20% chance of survival so there is significant emphasis on early diagnosis and treatment.

A number of grafts have been developed to treat a large aneurysm and to reduce the likelihood of rupture. For example EP 1325716 describes a three or four part graft which is assembled *in vivo.* This graft suffers from the need for complex assembly during surgery. WO 2004/002370 describes a one or two part endovascular graft having intermediate stents along its length.

Borst et al., (in Thorac Cardiovasc Surg (1983) 31:37-40) describes a technique commonly termed the "Elephant Trunk" technique to surgically treat aneurysm in the descending thoracic aorta. This technique has been demonstrated to reduce the risk of multiple stage aortic replacement (see Estrera et al., in Ann Thorac Surg (2002 74:S1803-5).

Roux et. al. (FR 2850008) describes a vascular prosthesis comprising an annular collar having a diameter substantially greater than that of the tubular body of the prosthesis. The collar can be trimmed to adapt to the diameter of a target blood vessel.

Quiachon et. al. (US 5957973) discloses an intraluminal grafting system comprising a balloon catheter assembly, a capsule catheter assembly, and a capsule jacket assembly. A vascular graft is manoeuvred into place using the system, and the system is withdrawn after expanding the graft attachment means and fixing the graft in position.

The "Elephant Trunk" graft is implanted into the patient in a first stage procedure in which the graft is used to replace the ascending aorta and aortic arch and a length of the graft is left hanging in the descending aorta. A second stage procedure involves extending the "Elephant Trunk" graft using a second vascular graft which is anastomosed distally to healthy aorta.

Since the technique requires a two stage process, there remains the possibility of aneurysm rupture before the second stage of the repair can be completed and various improvements to the technique have been suggested (see EP 1245202 and US 2004/0044395). This is especially of concern when the diameter of the aneurysm exceeds 5-6cm since there is an increased tension at the distal anastomosis due to the mismatch between the graft and the aorta. Although various approaches have been attempted to reduce this problem, a modified "Elephant Trunk" graft has proved to be most successful.

This modified graft (termed the "Dumbo" graft) is characterised by a 70mm sewing disc (for example of gelatine coated woven polyester) located around the main tube of the graft (see Neri et al., Ann Thorac Surg (2004) 78:E17-18). The sewing disc of the Dumbo graft enables repair of complex aortic lesions that involve both the aortic arch and the descending aorta, even in the presence of a size mismatch between the graft and the aorta at the level of the distal anastomosis. The sewing ring of the graft is able to cover the gap between the aorta and the graft, thus reducing tension on the sutures and avoiding the need to trim fragile tissues of the diseased aorta.

An endovascular device such as a stent is suggested for use to perform the second stage of the procedure. When performing the endovascular second stage, the distal aorta is not visible and the procedure relies on X-ray fluoroscopy. The endovascular device (for example a stent) is designed to be visible under X-ray and carries radio-opaque markers. However, it is important to establish the relative positions of the endovascular device and the previously inserted graft. Even if the second stage is performed surgically, it is useful to be able to identify the location of the distal end of the first stage graft (the Dumbo graft) to assist with planning of the second stage.

Neri et al., (supra) suggests marking the distal end of the first graft with metal clips, during the operation, for this purpose. These clips cannot be attached to the graft during manufacture, as the graft is trimmed at the time of implant to adjust its length to match the patient's anatomy. The position of the distal end is, therefore, not known until the time of implant.

We have now found that the problem of marking the distal end of the graft can be overcome by the inclusion of two diametrically opposed rows of spaced radio-opaque markers on the distal portion of the graft during manufacture.

Accordingly, the present invention provides an Elephant Trunk graft having two diametrically opposed rows of spaced radio-opaque markers on the distal portion thereof, as defined in claim 1.

The term "Elephant Trunk" graft refers to any vascular graft suitable to repair thoracic aortic aneurysm. The graft will normally replace the ascending aorta and arch of the aorta. In more detail the graft will include a 20 to 40mm (usually 25-35mm for example 30mm) diameter graft, optionally having a side branch attached thereto. The usual diameter of the side branch will be 5-15mm, typically 8-12mm, for example 10mm.

Optionally up to 4 branches can be present (to replace the supra aorta trunks).

In one embodiment the markers are disposed in pairs with one marker of each pair located on the upper (cranial) surface of the graft and the second marker of each pair being located on the lower (caudal) surface of the graft. There are thus two diametrically opposed rows of markers.

The spacing of the markers is selected to allow precise placement of the endovascular device (stent), whilst avoiding confusion caused by too many markers. We have found that a longitudinal spacing of 8-12mm (for example 10mm) between each markers is satisfactory.

In the present invention the Elephant Trunk graft includes a sewing ring collar (ie. is a Dumbo graft).

The material used to form the markers is chosen to be both biocompatible and highly visible under X-ray. Suitable materials include non-toxic metals such as gold, platinum or tantalum. Plastics rendered radio-opaque by the use of fillers such as barium sulphate or tungsten powder are also suitable.

The markers may be attached to the graft by sewing and advantageously the markers are sized and shaped to allow the passage of a sewing needle and thread. Preferred shapes include a ring or "doughnut" as well as button-like markers in the form of small plates with holes. Alternatively, the markers can be attached to the outer surface of the graft using adhesive bonding or welding. Optionally the markers could be formed into the body of the graft by incorporating metallic or other radio opaque yarns into the textile structure of the graft.

In use the surgeon would trim the graft to length by cutting between a pair of markers. The last remaining markers then identify the distal end of the graft and correct positioning of the endovascular device can be determined by checking the amount of overlap between the endovascular device and the graft. This is easily assessed by counting the markers from the distal end and advancing the endovascular device until it is inserted to lie between an appropriate pair of markers.

Thus, the present invention further provides a graft as described above for use in replacing diseased (eg. aneurysmal) tissue of the ascending aorta and aortic root. The graft can be trimmed to the required length immediately prior to implant, and the newly formed distal end of the graft will remain visible under X-ray to allow completion of the second graft procedure.

The present application will be now further described with reference to the figures in which:
Figure 1 shows a typical Elephant Trunk graft having radio-opaque markers attached to the distal end thereof; and
Figure 2 shows an enlarged view of the radio-opaque marker attached to the graft of Fig 1.

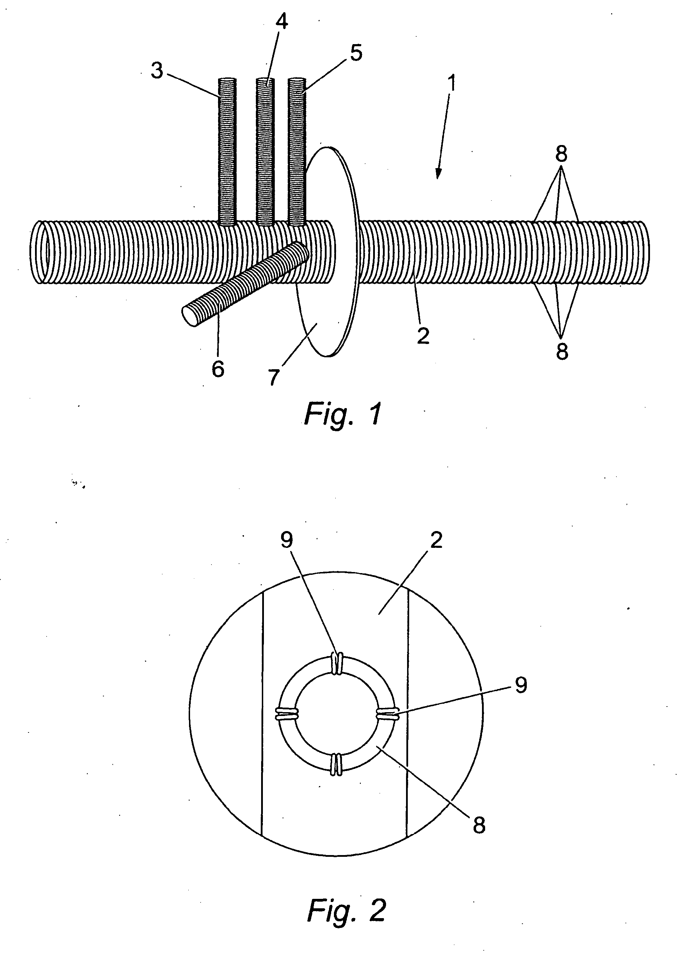

A typical Elephant Trunk graft 1 is shown in Fig 1 and comprises a main tubular body 2 and three side tubes 3, 4 and 5 which are used to connect to branches of the aorta in the patient. A further side tube 6 is used to allow access to the interior of the graft 1, in particular the lumen of tubular body 2. An endovascular device (stent) is generally introduced down the lumen of the tube 6 during the second stage of the procedure. A sewing ring 7 extends outwardly around the circumference of the tubular body 2 and is located approximately half way along the length of graft 1. Radio-opaque markers 8 are spacedly attached to the distal end of tubular body 2 with a typical spacing of 10mm between each marker. As illustrated the markers are attached to the outer surface of the graft. However in certain embodiments the markers could be attached to the inner surface of the graft.

As illustrated in Fig 1, the Elephant Trunk graft 1 has two diametrically opposed rows of markers 8 positioned on body 2. The markers extend along the distal end of the tubular body 2 approximately half way to the sewing ring 7. Suitably each row of markers comprises 4 to 16 markers, preferably 6 to 8 markers.

As shown in more detail in Fig 2 each of the radio-opaque markers 8 are sewn onto the outer portion of the main body 2 using 4 to 6 equispaced stitches 9. As illustrated, the radio opaque markers are formed from a metal such as tantalum or gold and are in a general doughnut shape. However, other forms of the markers 8 are also possible.

## Claims

1. An Elephant Trunk graft (1) comprising a sewing ring (7) for attachment to the aorta, **characterized in that** said graft further comprises two diametrically opposed rows of spaced radio-opaque markers (8) on the distal portion thereof, the length of the graft being adjustable by cutting and wherein the distal end of the graft after adjustment is identified by the last marker.

2. The graft (1) as claimed in Claim 1 having a space of 8 to 12mm between each marker (8) in a row.

3. The graft (1) as claimed in any one of Claims 1 or 2 wherein said radio-opaque markers (8) are formed from gold, platinum or tantalum.

4. The graft (1) as claimed in any one of Claims 1 to 3 wherein said radio-opaque markers (8) are sewn onto the graft.

5. The graft (1) as claimed in any one of Claims 1 to 4 for use in aortic aneurysm repair.

6. The graft (1) as claimed in any one of Claims 1 to 4 for use in replacing diseased tissue of the ascending aorta and aortic arch.

## Patentansprüche

1. Ein Elefantenrüsseltransplantat (1), das einen Nähring (7) zum Befestigen an der Aorta beinhaltet, **dadurch gekennzeichnet, dass** das Transplantat ferner zwei diametrisch gegenüberliegende Reihen von mit Abstand angeordneten röntgenstrahlenundurchlässigen Markierungen (8) auf dem distalen Abschnitt davon beinhaltet, wobei die Länge des Transplantats durch Schneiden anpassbar ist und wobei das distale Ende des Transplantats nach der Anpassung durch die letzte Markierung identifiziert wird.

2. Transplantat (1) gemäß Anspruch 1, das zwischen jeder Markierung (8) in einer Reihe einen Abstand von 8 bis 12 mm aufweist.

3. Transplantat (1) gemäß einem der Ansprüche 1 oder 2, wobei die röntgenstrahlendurchlässigen Markierungen (8) aus Gold, Platin oder Tantal gebildet sind.

4. Transplantat (1) gemäß einem der Ansprüche 1 bis 3, wobei die röntgenstrahlendurchlässigen Markierungen (8) auf das Transplantat genäht sind.

5. Transplantat (1) gemäß einem der Ansprüche 1 bis 4 zur Verwendung bei der Reparatur eines Aortenaneurysmas.

6. Transplantat (1) gemäß einem der Ansprüche 1 bis 4 zur Verwendung beim Ersetzen von krankem Gewebe der aufsteigenden Aorta und des Aortenbogens.

## Revendications

1. Une greffe en trompe d'éléphant (1) comprenant un anneau de couture (7) destiné à être attaché à l'aorte, **caractérisée en ce que** ladite greffe comprend de plus deux rangées diamétralement opposées de repères radio-opaques espacés (8) sur la portion distale de celle-ci, la longueur de la greffe pouvant être ajustée en la coupant et dans laquelle l'extrémité distale de la greffe après ajustement est identifiée par le dernier repère.

2. La greffe (1) telle que revendiquée dans la revendication 1 présentant un espace de 8 à 12 mm entre chaque repère (8) dans une rangée.

3. La greffe (1) telle que revendiquée dans n'importe laquelle des revendications 1 ou 2 dans laquelle lesdits repères radio-opaques (8) sont formés à partir d'or, de platine ou de tantale.

4. La greffe (1) telle que revendiquée dans n'importe laquelle des revendications 1 à 3 dans laquelle lesdits repères radio-opaques (8) sont cousus sur la greffe.

5. La greffe (1) telle que revendiquée dans n'importe laquelle des revendications 1 à 4 destinée à être utilisée dans la réparation d'anévrisme aortique.

6. La greffe (1) telle que revendiquée dans n'importe laquelle des revendications 1 à 4 destinée à être utilisée pour remplacer du tissu malade de l'aorte ascendante et de la crosse aortique.
